(19) Europäisches Patentamt
European Patent Office
Office européen des brevets

(11) Publication number: **0 405 956 A1**

## (12) EUROPEAN PATENT APPLICATION

(21) Application number: 90307051.4

(22) Date of filing: 27.06.90

(51) Int. Cl.5: **C07C 29/136**, C07C 31/22

(30) Priority: 29.06.89 GB 8914991

(43) Date of publication of application:
**02.01.91 Bulletin 91/01**

(84) Designated Contracting States:
**AT BE CH DE DK ES FR GB GR IT LI LU NL SE**

(71) Applicant: BP Chemicals Limited
Belgrave House 76 Buckingham Palace Road
London, SW1W 0SU(GB)

(72) Inventor: Gracey, Benjamin Patrick
BP Chemicals Limited
Salt End, Hull HU12 8DS(GB)

(74) Representative: Krishnan, Suryanarayana
Kalyana et al
BP INTERNATIONAL LIMITED Patents &
Agreements Division Chertsey Roadoad
Sunbury-on-Thames Middlesex TW16
7LN(GB)

(54) Hydrogenation of alpha-hydroxy ketones.

(57) This invention relates to a process for the catalytic hydrogenation of alpha-hydroxy ketones to the corresponding alcohol in a heterogeneous liquid phase reaction medium which contains a carboxylic acid. The carboxylic acid may be a simple carboxylic acid or a hydroxy carboxylic acid.

The presence of the carboxylic acid improves both the yield of and selectivity to the alcohol product.

## HYDROGENATION OF ALPHA-HYDROXY KETONES

The present invention relates to an improved process for the hydrogenation of alpha-hydroxy ketones, especially dihydroxy acetone, to the corresponding alcohols.

It is well known to hydrogenate ketones to the corresponding alcohols using a homogeneous or heterogeneous catalyst system. US-A-4024193, describes amongst others, the hydrogenation of pure dihydroxy acetone to glycerol using a homogeneous catalyst system.

Again our published EP-A-306215 describes a process for synthesising glycerol by hydrogenation of dihydroxy acetone formed by self-condensation of formaldehyde. In this document both homogeneous and heterogeneous hydrogenation techniques are described.

The aforementioned process generally involves the use of a solvent, usually an alcohol, for the hydrogenation.

It has now been found, however, that the selectivity of this reaction to the desired alcohol product can be significantly increased by carrying out the hydrogenation reaction in the presence of a carboxylic acid.

Accordingly the present invention relates to a process for the hydrogenation of alpha-hydroxy ketones to the corresponding alcohols in the presence of a heterogeneous hydrogenation catalyst and hydrogen in a liquid reaction medium characterised in that the hydrogenation is carried out in the presence of a carboxylic acid.

An example of the alpha-hydroxy ketone that may be hydrogenated in this manner is dihydroxy acetone which gives rise to glycerol.

The alpha-hydroxy ketone may be derived from any source e.g. from the self-condensation of or cross-condensation of an aldehyde or mixture of aldehydes respectively. One such method is described in our published EP-A-306215. Another method of synthesis is described in J.A.C.S., 1984, vol. 106, pp4829-4832 by Matsumoto, T. et al.

A further method synthesising alpha-hydroxy ketones is described in EP-A-245976.

The alpha-hydroxy ketone is hydrogenated with hydrogen in the presence of a heterogeneous catalyst to the corresponding alcohol.

During the synthesis of alpha-hydroxy ketones such as dihydroxy acetone, a by-product of the process is eruthrulose hydrate. The latter can also be hydrogenated by the process of the present invention to methyl glycerol, a product very similar to glycerol.

The heterogeneous hydrogenation catalyst is suitably a finely divided or a supported metal. For example such a catalyst may be a Group VIII metal e.g. nickel, Raney nickel or ruthenium supported on a support inert under the reaction conditions e.g. carbon or graphite, or a copper chromite type catalyst.

Hydrogen is readily available on a commercial scale. It may be used in a commercially available form or may, if desired, be further purified. The hydrogen partial pressure may suitably be in the range from 10 to 30,000 Kpa, preferably from 100 to 10,000 KPa.

The hydrogenation step may suitably be accomplished at elevated temperature, suitably in the range from ambient to 150° C, preferably from 40 to 150° C, most preferably from 40 to 120° C.

The liquid reaction medium for the hydrogenation step is suitably a solvent capable of dissolving the hydrogenation reactants. Suitable solvents include, but are not restricted to, alcohols, water, ethers and mixtures of one or more of these.

The reaction medium is preferably an aqueous medium containing at least 40%w/w of water. The reaction medium may contain $C_1$-$C_6$ aliphatic alcohols such as the butanols and methyl pentanols and also glycerol product either from an earlier hydrogenation reaction or added as such from an external source.

A feature of the present invention is that the hydrogenation is carried out in the presence of a carboxylic acid. The carboxylic acid is preferably an aliphatic carboxylic acid and is more preferably a $C_1$-$C_4$ saturated aliphatic carboxylic acid or hydroxy carboxylic acid. Acetic acid, lactic acid and citric acid are most preferred. Moreover, this improvement can be observed even in the case of e.g. dihydroxy acetone contaminated with small amounts of formaldehyde. This contaminant, which can be present if dihydroxy acetone is produced by self condensation of formaldehyde, considerably supresses selectivity to glycerol upon hydrogenation of dihydroxy acetone. However, addition of acetic acid to the hydrogenation step restores selectivity to glycerol as if formaldehyde contaminant were absent.

The carboxylic acid is suitably present in the liquid hydrogenation reaction medium in an amount so as to maintain in the pH of the reaction system in the range of 2-6, preferably from 2.9-5.5.

The hydrogenation step may suitably be carried out batchwise or continuously, preferably continuously.

For batch operation the duration of the hydrogenation reaction will vary with the type and concentration of the hydrogenation catalyst, the hydrogen partial pressure and with the nature of the product being hydrogenated, i.e. whether crude or pure or whether the reaction is carried out in situ. The glycerol product formed upon hydrogenation of e.g. dihydroxy acetone can be purified and recovered by methods well known in the art. A suitable method of purification is vacuum distillation. If required post treatments known to those skilled in the art may be used. Such treatments include, but are not restricted to, passage over a carbon bed and treatment with a bleaching agent.

The present process is clearly simpler and less expensive to operate than the synthetic processes used hitherto and gives a much higher selectivity to the desired alcohol product. The raw materials are readily available and the products separated and purified.

The present invention is further illustrated with reference to the following Examples.

Examples 1 and Comparative Test 1

A 300ml stainless steel autoclave was purged with nitrogen and then charged with catalyst (64%w/w Ni on a support containing alumina sold as Harshaw NI5132P - 1g) and an appropriate solution of reactants, sealed and repeatedly pressurised and depressurised with hydrogen until a pressure of 10,000 KPa was attained. In Example 1 and Comparative Test 1 this reactant solution consisted of 8% DHA in deionised water. 0.4g of acetic acid was added to the charge used for Example 1 which then had a pH of 2.96.

Heating and stirring was commenced (5°C/min, 1100rpm) until 110°C was attained. This temperature was maintained for 4.5 hours with a continued stirring. After this period the heating/stirring was ceased and the autoclave allowed to cool to room temperature. The liquid product was sampled and analysed by high performance liquid chromatography (HPLC). The results are tabulated in Table 1 below.

In Comparative Test 1, which had no added carboxylic acid, selectivity to glycerol was 76.4%. In Example 1 the presence of acetic acid increased this selectivity to 97.3%.

Examples 2-7

A 300ml stainless steel autoclave was purged with nitrogen and then charged with catalyst (54%w/w Ni on a support containing alumina sold as Sudchemie G134 - 1g) and an appropriate reactant solution as shown in Table 2 below. A combination of citric acid and sodium citrate was used to obtain the pH specified. The autoclave was then sealed and repeatedly pressurised with hydrogen to purge out nitrogen. Finally the autoclave was pressurised to 25 barg with hydrogen.

Heating and stirring was commenced (5°C/min, 1000 rpm) until a temperature of 110°C was obtained. This temperature was maintained for 3 hours with continued stirring. After this period the heating/stirring was ceased and the autoclave allowed to cool to room temperature. The liquid product was analysed as in Example 1. Table 2, tabulates the individual feed compositions and the experimental results. These results demonstrate that the presence of citric acid is beneficial for the hydrogenation of dihydroxyacetone with Sudchemie G134 catalyst.

Examples 8-10

The experimental procedure used in all these Examples was as in Example 1 above except for the reactant quantities specified. The compositions and reaction conditions used and the results achieved are tabulated in Table 3 below.

These results demonstrate the effect of adding carboxylic acids to the reaction medium during hydrogenation of dihydroxyacetone with a Harshaw Ni 5132 catalyst.

Examples 11-17 and Comparative Tests 2 and 3

The experimental procedure used in all these Examples and Comparative Tests was the same as that used in Example 1 above except for the reactant quantities specified. The compositions and the reaction conditions used and the results achieved are tabulated in Tables 4 and 5 below.

The results in Comparative Test 2 and Example 11 show that the pH adjustment using carboxylic acids according to the present invention is beneficial even in the presence of formaldehyde as an impurity during the hydrogenation reaction.

3

TABLE 1

| THE EFFECT OF ACETIC ACID ON THE HYDROGENATION OF DHA IN WATER AT 10,000 KPa | | | | | | |
|---|---|---|---|---|---|---|
| NO. | pH | WT% DHA FINAL | WT% GLYCEROL FINAL | SELECTIVITY TO GLYCEROL | CONVERSION OF DHA | CH₃COOH added (G) |
| COMPARATIVE TEST 1 | 6.5 | 0.1 | 6.1 | 76.42 | 98.75 | 0.0 |
| EXAMPLE 1 | 2.96 | 0.1 | 7.8 | 97.39 | 98.75 | 0.4 |

DHA - DIHYDROXY ACETONE

AUTOCLAVE CHARGE: 8% DHA IN DEIONISED WATER (92g FOR COMPARATIVE TEST 1 AND 91.6g FOR EXAMPLE 1), 1 g CATALYST (HARSHAW NI 5132P).

CONDITIONS: 10,000 KPa H₂, 110°C, 1100rpm, 4.5 HOURS.

TABLE 2

EFFECT OF CITRIC ACID ON SELECTIVITY OF DIHYDROXYACETONE HYDROGENATION TO GLYCEROL

| Example Number | FEED | | |
|---|---|---|---|
| | pH of water solution at 20°C | Buffered water(g) | Dihydroxyacetone (g) |
| 2 | 2 | 180 | 20 |
| 3 | 3 | 180 | 20 |
| 4 | 4 | 180 | 20 |
| 5 | 5 | 180 | 20 |
| 6 | 6 | 180 | 20 |
| 7 | 7 | 180 | 20 |

PRODUCT ANALYSIS

| Example Number | Feed Dihydroxyacetone (%w/w) | Glycerol (%w/w) |
|---|---|---|
| 2 | 0 | 0.4 |
| 3 | 0 | 1.5 |
| 4 | 0 | 1.8 |
| 5 | 0 | 0.7 |
| 6 | 0 | 0.6 |
| 7 | 0 | 0.0 |

TABLE 3

| THE EFFECT OF ACETIC ACID ON THE HYDROGENATION OF DHA TO GLYCEROL | | | | | | | |
|---|---|---|---|---|---|---|---|
| Example Number | WT% DHA Initial | WT% DHA Final | WT% GLYCEROL Final | % Sel to Glycerol | % Conv of DHA | WT% Acetic Acid Added | pH of Solution |
| 8 | 16 | 0.0 | 15.1 | 94.40 | 100.00 | 0.30 | 2.96 |
| 9 | 24 | 0.0 | 21.3 | 88.80 | 100.00 | 0.40 | 2.96 |
| 10 | 32 | 0.4 | 27.9 | 87.20 | 98.60 | 1.58 | 2.96 |
| DHA = Dihydroxyacetone | | | | | | | |
| P = 100 barg, CAT = 1g (HARSHAW Ni 5132P) 110° C, 1000rpm, 4.5 HOURS. | | | | | | | |

TABLE 4

| THE EFFECT OF ACETIC ACID ON THE SELECTIVITY OF THE HYDROGENATION OF DHA TO GLYCEROL | | | | | | | | |
|---|---|---|---|---|---|---|---|---|
| Example Number | Catalyst | WT% DHA Initial | WT% DHA Final | WT% GLYCEROL Final | % Sel to Glycerol | % Conv of DHA | WT% Acetic Acid Added | WT% CH2O Added |
| CT 2 | 5%Ru/C | 8 | 0 | 2.7 | 33.8 | 100 | 0.00 | 2 |
| 11 | 5%Ru/C | 8 | 0 | 4.5 | 56.3 | 100 | 0.41 | 2 |
| CT 3 | 5%Ru/CH19C | 50 | 0 | 6.6 | 13.2 | 100 | 0.00 | 0 |
| 12 | 5%Ru/CH19C | 50 | 0 | 47.8 | 95.6 | 100 | 0.16 | 0 |
| 13 | 5%Ru/CH19A | 50 | 0 | 49.6 | 99.2 | 100 | 0.16 | 0 |
| DHA = Dihydroxyacetone | | | | | | | | |
| CT = Comparative Test (not according to the invention) | | | | | | | | |
| P = 100 barg, CAT = 1g, 110° C, 1000rpm, 4.5 HOURS. | | | | | | | | |
| 5%Ru/C Ex Aldrich | | | | | | | | |
| 5%Ru/CH19A Ex Johnson Matthey | | | | | | | | |
| 5%Ru/CH19C Ex Johnson Matthey | | | | | | | | |

TABLE 5

| THE EFFECT OF DIFFERENT CONCS OF ACETIC ACID ON THE SELECTIVITY OF THE HYDROGENATION OF DHA TO GLYCEROL | | | | | | | |
|---|---|---|---|---|---|---|---|
| Example Number | Catalyst | WT% DHA Initial | WT% DHA Final | WT% GLYCEROL Final | % Sel to Glycerol | % Conv of DHA | WT% Acetic Acid Added |
| 14 | 5%Ru/CH19A | 50 | 0 | 26.1 | 52.2 | 100 | 1.00 |
| 15 | 5%Ru/CH19A | 50 | 0 | 48.2 | 96.4 | 100 | 0.16 |
| 16 | 5%Ru/CH19C | 50 | 0 | 28.2 | 56.4 | 100 | 2.00 |
| 17 | 5%Ru/CH19C | 50 | 0 | 47.8 | 95.6 | 100 | 0.16 |

DHA = Dihydroxyacetone

P = 100 barg, CAT = 1g, 110° C, 1000rpm, 4.5 HOURS.

5%Ru/CH19A Ex Johnson Matthey

5%Ru/CH19C Ex Johnson Matthey

**Claims**

1. A process for the hydrogenation of alpha-hydroxy ketones to the corresponding alcohols in the presence of a heterogeneous hydrogenation catalyst and hydrogen in a liquid reaction medium characterised in that the hydrogenation is carried out in the presence of a carboxylic acid.

2. A process according to Claim 1 wherein the alpha-hydroxy ketone is dihydroxyacetone and/or eruthrulose and the corresponding alcohol is glycerol and/or methyl glycerol respectively.

3. A process according to Claim 1 or 2 wherein the hydrogenation catalyst is selected from a Group VIII metal supported on a support which is inert under the reaction conditions, and a copper chromite type catalyst.

4. A process according to Claim 3 wherein the catalyst is Raney nickel, Harshaw nickel on alumina or ruthenium on carbon.

5. A process according to any one of the preceding Claims wherein the hydrogenation is carried out at hydrogen partial pressures from 10-30000 KPa and a temperature from ambient to 150° C.

6. A process according to any one of the preceding Claims wherein the carboxylic acid is a $C_1$ to $C_4$ saturated aliphatic carboxylic acid or hydroxy carboxylic acid.

7. A process according to Claim 6 wherein the carboxylic acid is acetic acid, lactic acid or citric acid.

8. A process accoridng to any one of the preceding Claims wherein the carboxylic acid is present in the liquid medium in an amount so as to maintain the pH of the reaction system in the range from 2-6.

9. A process according to any one of the preceding Claims wherein the liquid reaction medium comprises at least 40% w/w of water.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP-A-0 306 215 (BP CHEMICALS LTD) <br> * Claims * | 1-3,5 | C 07 C 29/136 <br> C 07 C 31/22 |
| Y | GB-A-1 222 216 (ICI LTD) <br> * Claims * | 1,5-7 | |
| Y | CHEMICAL ABSTRACTS, vol. 90, no. 1, 1st January 1979, page 539, abstract no. 5845x, Columbus, Ohio, US; T.A. ANTONOVA et al.: "Catalytic hydrogenation of dihydroxyacetone", & SB. TR. VSES. ZAOCHN. POLITEKH. INST. 1976, 100, 7-14 <br> * Abstract * | 1-5 | |

TECHNICAL FIELDS SEARCHED (Int. Cl.5)

C 07 C 29/00
C 07 C 31/00

The present search report has been drawn up for all claims

| Place of search | Date of completion of the search | Examiner |
|---|---|---|
| THE HAGUE | 02-10-1990 | BONNEVALLE E.I.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)